# EUROPEAN PATENT APPLICATION

(11) **EP 4 349 809 A1**
(43) Date of publication of application: **10.04.2024**
(21) Application number: 22833711.9
(22) Date of filing: 01.07.2022
(51) Int. Cl.: C07D 209/04, C07D 209/14, C07D 403/04, A61K 31/404

(54) **METHOD FOR PREPARING INTERMEDIATE FOR SYNTHESIS OF XANTHINE OXIDASE INHIBITOR**

(30) Priority: 02.07.2021 KR 20210087042
(71) Applicant: LG Chem, Ltd., Seoul 07336 (KR)
(72) Inventor: LEE, Seok Ju, Seoul 07796 (KR); PARK, Ah Byeol, Seoul 07796 (KR); LEE, Ju Yeol, Seoul 07796 (KR); KIM, Ki Dae, Seoul 07796 (KR); JEONG, Hui Rak, Seoul 07796 (KR)
(74) Representative: Hoffmann Eitle
(86) International application number: PCT/KR2022/009544
(87) International publication number: WO 2023/277658

(57) **Abstract**

The present invention relates to a method for preparing an intermediate for synthesis of a xanthine oxidase inhibitor and, more specifically, to a method for preparing compounds of chemical formulas 2 and 4 by using low-priced starting materials and ligands and employing chelating extraction and purification techniques.

## Description

### TECHNICAL FIELD

The present invention relates to methods of preparing intermediates for synthesizing xanthine oxidase inhibitor. More specifically, the present invention relates to methods for preparing compounds of the following Formula 2 and Formula 4 by using inexpensive starting materials and ligands, and a purification technique utilizing chelating extraction: 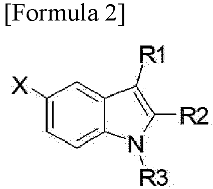 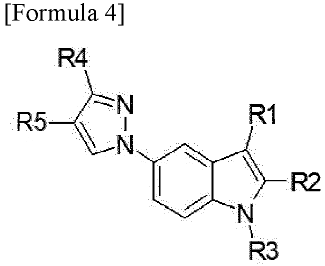 wherein,
X is F, Cl, Br or I;
R1 is hydrogen or CN;
R2 is hydrogen, halogen, C₁-C₇ alkyl, C₁-C₇ alkoxy-C₁-C₇ alkyl or phenyl;
R3 is hydrogen; C₁-C₇ alkyl unsubstituted or substituted by one or more substituents selected from halogen, C₃-C₇ cycloalkyl and O-R6; C₃-C₇ cycloalkyl; or 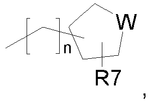 wherein R6 is C₁-C₄ alkyl, W is O or S, R7 is hydrogen or C₁-C₄ alkyl, and n is an integer of 0 to 3;
R4 is hydrogen, halogen or C₁-C₇ alkyl; and
R5 is -C(O)OR8, wherein R8 is hydrogen, C₁-C₇ alkyl or C₃-C₇ cycloalkyl.

### BACKGROUND ART

Xanthine oxidase is known as an enzyme which converts hypoxanthine to xanthine and further converts thus-formed xanthine to uric acid. Although most mammals have uricase, humans and chimpanzees do not, thereby uric acid is known to be the final product of purine metabolism (S. P. Bruce, Ann. Pharm., 2006, 40, 2187-2194). Sustained elevation of blood concentration of uric acid causes various diseases, representatively including gout.

As described above, gout is caused by an elevated level of uric acid in the body, indicating the condition in which uric acid crystals accumulated in cartilage, ligament and surrounding tissue induce severe inflammation and pain. Gout is a kind of inflammatory articular disease, and its incidence rate has steadily increased during past 40 years (N. L. Edwards, Arthritis & Rheumatism, 2008, 58, 2587-2590).

Accordingly, various studies have been conducted to develop new xanthine oxidase inhibitors, and Korean Patent Application Publication No. 10-2011-0037883 discloses a novel compound of the following formula, which is effective as a xanthine oxidase inhibitor:

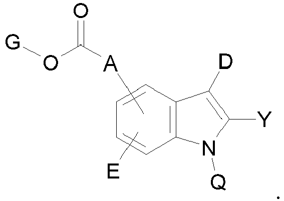

In Korean Patent Application Publication No. 10-2011-0037883 disclosing a conventional preparation step of 5-bromo-3-cyano-1-isopropyl-indole-which is an intermediate of the xanthine oxidase inhibitor, CsCO₃-which is inconvenient to use due to its low economic effectiveness and high density-was used, so that it was necessary to secure a substitute for it. In addition, since there is no established purification method, the organic layer obtained after the reaction and work-up process was concentrated and the next reaction was carried out immediately. At this time, there was a problem in that 5-bromo-3-cyano-1-isopropyl-indole included in the reaction mixture because it was not purified acts as a causative material for generating impurity in the next reaction, adversely affecting product quality.

Furthermore, the conventional preparation step for 1-(3-cyano-1-isopropyl-indol-5-yl)pyrazole-4-carboxylic acid ethyl ester has a very long reaction time of 35 to 48 hours, and the starting material, 5-bromo-3-cyano-1-isopropyl-indole, remains and various impurities are generated. As a result, such materials can affect raw material medicine, so that it was necessary to improve the quality and yield. In addition, after completion of the reaction for 1-(3-cyano-1-isopropyl-indol-5-yl)pyrazole-4-carboxylic acid ethyl ester, Na₂SO₄ and silica gel were put into the reactor and then the adsorption process was carried out to remove Cu-complex and in-organic by-products in the work-up process. At this time, there is an issue of cleaning the manufacturing equipment. In addition, because filtration and washing to remove adsorbents and solid complexes lengthened the process time, and solid waste treatment was difficult, it is necessary to develop an effective purification method.

### [Prior Document]

Patent Document: Korean Patent Application Publication No. 10-2011-0037883

### DISCLOSURE OF INVENTION

### TECHNICAL PROBLEM

Accordingly, the technical problem of the present invention is the provision of novel methods for preparing compounds of Formula 2 and Formula 4, which are key intermediates in the synthesis of xanthine oxidase inhibitors, at a lower cost, effectively reducing residual impurity, shortening reaction time, improving yield and eliminating the possibility of generating solid waste: 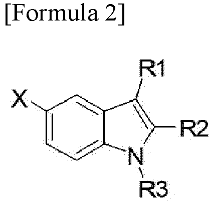 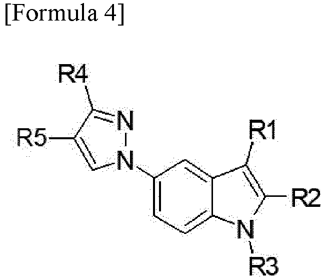 wherein X, R1, R2, R3, R4 and R5 are the same as defined herein.

### SOLUTION TO PROBLEM

To solve the above technical problem, there is provided a method for preparing a compound of the following Formula 2, comprising:
i) a step of reacting a compound of Formula 1 and X-R3 with a base comprising KOH in an organic solvent; and
ii) a step of crystallizing a product obtained in step (i) by using alcohol and an anti-solvent comprising hydrocarbon having 5 to 8 carbon atoms: 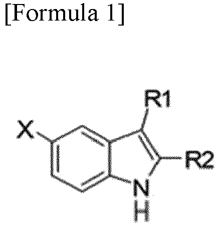 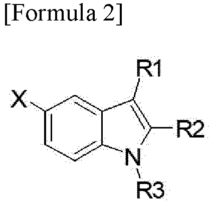
wherein,
X is F, Cl, Br or I;
R1 is hydrogen or CN;
R2 is hydrogen, halogen, C₁-C₇ alkyl, C₁-C₇ alkoxy-C₁-C₇ alkyl or phenyl; and
R3 is hydrogen; C₁-C₇ alkyl unsubstituted or substituted by one or more substituents selected from halogen, C₃-C₇ cycloalkyl and O-R6; C₃-C₇ cycloalkyl; or 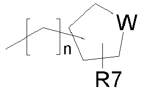 , wherein R6 is C₁-C₄ alkyl, W is O or S, R7 is hydrogen or C₁-C₄ alkyl, and n is an integer of 0 to 3.

According to one embodiment of the present invention, X-R3 may be 2-iodopropane, but is not limited thereto.

According to one embodiment of the present invention, the organic solvent may be acetone, but is not limited thereto.

According to one embodiment of the present invention, the alcohol may be one or more selected from the group consisting of methanol, ethanol, propanol, isopropanol and butanol, but is not limited thereto.

According to one embodiment of the present invention, the hydrocarbon having 5 to 8 carbon atoms may be selected from the group consisting of hexane, heptane and a mixture thereof, but is not limited thereto.

According to one embodiment of the present invention, R1 may be CN, R2 may be hydrogen, and R3 may be isopropyl, but is not limited thereto.

According to another aspect of the present invention, there is provided a method for preparing a compound of the following Formula 4, comprising:
a step of carrying out a C-N coupling reaction of a compound of Formula 2 and a compound of Formula 3 with a copper catalyst, a base and a ligand comprising N,N-dimethylethylenediamine in an organic solvent: 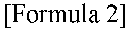 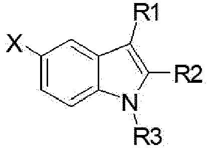 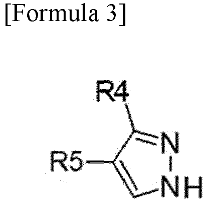 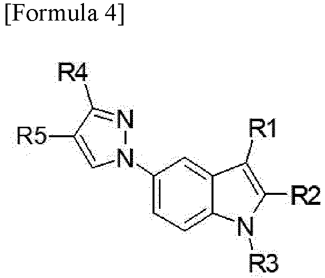
wherein,
X is F, Cl, Br or I;
R1 is hydrogen or CN;
R2 is hydrogen, halogen, C₁-C₇ alkyl, C₁-C₇ alkoxy-C₁-C₇ alkyl or phenyl;
R3 is hydrogen; C₁-C₇ alkyl unsubstituted or substituted by one or more substituents selected from halogen, C₃-C₇ cycloalkyl and O-R6; C₃-C₇ cycloalkyl; or 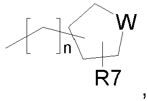 wherein R6 is C₁-C₄ alkyl, W is O or S, R7 is hydrogen or C₁-C₄ alkyl, and n is an integer of 0 to 3;
R4 is hydrogen, halogen or C₁-C₇ alkyl; and
R5 is -C(O)OR8, wherein R8 is hydrogen, C₁-C₇ alkyl or C₃-C₇ cycloalkyl.

According to one embodiment of the present invention, the organic solvent may be one or more selected from the group consisting of xylene, toluene, dimethylformamide (DMF) and dimethyl sulfoxide (DMSO), but is not limited thereto.

According to one embodiment of the present invention, the copper catalyst may be one or more selected from the group consisting of CuI, Cu(OAc)₂, Cu, Cu₂O and CuO, but is not limited thereto.

According to one embodiment of the present invention, the base may be one or more selected from the group consisting of potassium carbonate, cesium carbonate, potassium phosphate tribasic, triethylamine and sodium tert-butoxide, but is not limited thereto.

According to one embodiment of the present invention, the method for preparing a compound of Formula 4 may further comprise a step of purifying the compound of Formula 4 by using:
one or more chelating agents selected from the group consisting of EDTA, citric acid, potassium citrate and sodium citrate; and
one or more ligand reagents selected from the group consisting of ammonium chloride and aqueous ammonia, but is not limited thereto.

According to one embodiment of the present invention, R1 may be CN, R2 may be hydrogen, R3 may be isopropyl, R4 may be hydrogen, and R5 may be ethoxycarbonyl (-C(O)OEt), but is not limited thereto.

### EFFECTS OF THE INVENTION

The preparation method according to the present invention can secure economic effectiveness and effectively reduce residual impurity by replacing expensive Cs₂CO₃ with remarkably inexpensive KOH in the preparation of the compound of Formula 2. In addition, the preparation method according to the present invention dramatically shortens the reaction time in the preparation of the compound of Formula 4, and at the same time dramatically improves the yield compared to the conventional method and significantly reduces the possibility of generating solid waste, thereby making it easier to scale up.

### BRIEF DESCRIPTION OF DRAWINGS

Figure 1 is a comparison of HPLC peak areas of residual 5-bromo-3-cyano-1H-indole before and after crystallization of 5-bromo-3-cyano-1-isopropyl-indole. (GD40: 5-bromo-3-cyano-1H-indole, GD60: 5-bromo-3-cyano-1-isopropyl-indole)
Figure 2 represents the structures of the ligands subjected to the reaction of 1-(3-cyano-1-isopropyl-indol-5-yl)pyrazole-4-carboxylic acid ethyl ester.
Figure 3 represents the HPLC conversion ratio (%) of 5-bromo-3-cyano-1-isopropyl-indole into 1-(3-cyano-1-isopropyl-indol-5-yl)pyrazole-4-carboxylic acid ethyl ester.
Figure 4 represents the HPLC conversion ratio (%) of 5-bromo-3-cyano-1-isopropyl-indole into 1-(3-cyano-1-isopropyl-indol-5-yl)pyrazole-4-carboxylic acid ethyl ester.
Figure 5 represents the HPLC conversion ratio (%) of 5-bromo-3-cyano-1-isopropyl-indole into 1-(3-cyano-1-isopropyl-indol-5-yl)pyrazole-4-carboxylic acid ethyl ester.

### MODE FOR THE INVENTION

Hereinafter, the present invention is explained in more detail with the following examples. However, it must be understood that the protection scope of the present invention is not limited to the examples.

### Example 1: Results of 5-bromo-3-cyano-1-isopropyl-indole reaction test

### Example 1.1: Base screening of 5-bromo-3-cyano-1-isopropyl-indole

Cs₂CO₃, which is a raw material used for the synthesis of 5-bromo-3-cyano-1-isopropyl-indole in the previously known process, is quite expensive, and 1.7 equivalents thereof in respect to 5-bromo-3-cyano-1H-indole were added to the reaction and accounted for about 1% of the cost of raw materials. In addition, due to the high density of Cs₂CO₃ (density: 4.07 g/cm³), the agitation speed operation range was increased for smooth stirring during the reaction. In order to solve such problems, 5-bromoindole was first selected as an indole source and screening for various bases was carried out in the following Reaction Scheme 1 (Table 1).

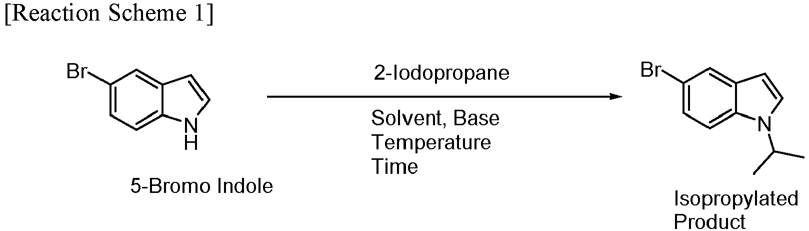

**[Table 1] Isopropylation screening experiment of 5-bromoindole**

| Run | Base (equiv) | Solvent (fold) | Temp. (°C) | Time (h) | Result (HPLC % ratio) | | | |
|---|---|---|---|---|---|---|---|---|
| | | | | | 16 min (SM) | 18 min | 20 min | 23 min (Prt) |
| Example 1-1-1 | Cs₂CO₃ (1.7) | DMF (10) | 25 | 2.5 | 52.5 | 8.6 | 2.1 | 36.6 |
| Example 1-1-2 | K₂CO₃ (1.7) | | | | 96.2 | 3.6 | 0.2 | - |
| Example 1-1-3 | STP (1.7) | | | | 41.3 | - | 2.5 | 56.2 |
| Example 1-1-4 | NaOMe (1.7) | | | | 50.2 | - | 1.3 | 48.5 |
| Example 1-1-5 | KOH (1.7) | | | | 15.4 | - | 2.0 | 82.6 |
| Example 1-1-6 | Cs₂CO₃ (1.7) | DMF (6) | | 6.0 | 61.8 | - | 2.5 | - |
| Example 1-1-7 | Cs₂CO₃ (1.7) | DMF (10) | 90 | 5.0 | 59.2 | - | 2.3 | 38.5 |
| Example 1-1-8 | K₂CO₃ (1.7) | | | | 74.0 | 0.7 | 1.1 | 24.1 |
| Example 1-1-9 | STP (1.7) | | | | 37.6 | - | 2.4 | 59.9 |
| Example 1-1-10 | NaOMe (1.7) | | | | 16.6 | - | 1.6 | 81.8 |
| Example 1-1-11 | KOH (1.7) | | | | 16.0 | - | 1.9 | 81.9 |
| Example 1-1-12 | KOH (2.0) | DMF (10) | 35 | 3.5 | 13.7 | - | 1.65 | 84.1 |
| Example 1-1-13 | KOH (2.0) | | | | 8.3 | - | 1.7 | 90.0 |
| Example 1-1-14 | KOH (2.4) | | | | 14.0 | 0.8 | 1.1 | 83.8 |
| Example 1-1-15 | KOH (2.4) | | | | 1.45 | 1.1 | 0.6 | 96.9 |
| Example 1-1-16 | NaOMe (2.0) | | 90 | 6.0 | 53.1 | 1.9 | 2.1 | 42.9 |
| Example 1-1-17 | NaOMe (2.0) | | | | 63.0 | 2.7 | 1.7 | 32.6 |
| Example 1-1-18 | NaOMe (2.4) | | | | 54.0 | - | 2.2 | 43.8 |

| | | | | | | | | |
|---|---|---|---|---|---|---|---|---|
| STP: sodium tert-pentoxide | | | | | | | | |

1.7 Equivalents of 2-iodopropane and DMF as the reaction solvent were selected, and a suitable base in terms of the ratio of conversion to the alkylated product was predicted while changing the reaction temperature and reaction time. In Examples 1-1-1 to 1-1-5, KOH showed the highest conversion ratio. In the case of Examples 1-1-7 to 1-1-11, after raising the reaction temperature to 90°C, the reaction was performed. As a result, the conversion ratio tendency of K₂CO₃ < Cs₂CO₃ < STP < NaOMe ≤ KOH can be known.

In Examples 1-1-12 to 1-1-18, the reaction temperature and equivalent were changed in order to compare KOH and NaOMe. As a result, when KOH was used as a base, the most desirable results were obtained. Based on the experiment in Table 1, an isopropylation reaction was carried out in the following Reaction Scheme 2 using 5-bromo-3-cyano-1H-indole in which CN group is substituted at the C3 position of 5-bromoindole (Table 2).

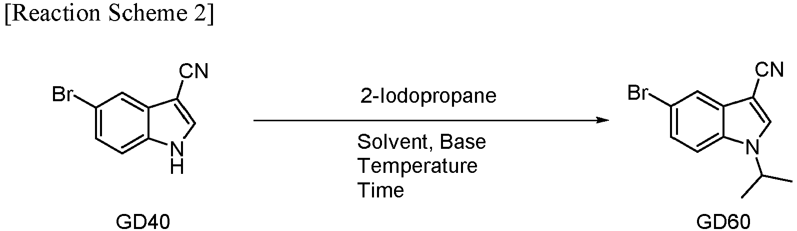

**[Table 2] 5-Bromo-3-cyano-1H-indole isopropylation screening experiment I**

| Run | 2-Iodo propane (equiv) | Base (equiv) | Solvent (fold) | Temp. (°C) | Time (h) | Result (HPLC % ratio) | | | |
|---|---|---|---|---|---|---|---|---|---|
| | | | | | | 10 min | 14 min (GD40) | 16 min | 18 min (GD60) |
| Example 1-2-1 | 1.7 | Cs₂CO₃ (1.7) | Acetone (5) | 60 | 3.0 | 1.11 | N/D | 2.17 | 95.06 |
| Example 1-2-2 | 1.7 | KOH (1.7) | DMF (7) | r.t | 5.0 | 0.83 | 18.10 | 2.55 | 78.42 |
| Example 1-2-3 | 2.2 | KOH (1.7) | | r.t | 4.0 | 0.93 | 11.68 | 2.86 | 84.37 |
| Example 1-2-4 | 1.7 | KOH (1.7) | | 154 | 7.0 | 0.43 | 7.40 | 1.78 | 89.75 |
| Example 1-2-5 | 1.7 | KOH (1.7) | Acetone (7) | 60 | 4.6 | 1.24 | N/D | 1.89 | 95.45 |

| | | | | | | | | | |
|---|---|---|---|---|---|---|---|---|---|
| GD40: 5-Bromo-3-cyano-1H-indole GD60: 5-Bromo-3-cyano-1-isopropyl-indole | | | | | | | | | |

Through Example 1-2-1, it was confirmed that after completion of the reaction under the reaction conditions of 5-bromo-3-cyano-1-isopropyl-indole, 5-bromo-3-cyano-1H-indole does not remain. In Examples 1-2-2 to 1-2-4, Cs₂CO₃ was replaced with KOH, and the reaction was carried out in a DMF solvent. As a result of changing the reaction temperature, and the equivalents of 2-iodopropane and KOH, the reaction proceeded well, but it was confirmed that 0.43 - 0.93% of the starting material (5-bromo-3-cyano-1H-indole) remained. In Example 1-2-5, KOH was used as the base, the reaction solvent was changed from DMF to acetone, and the reaction was carried out under reflux conditions. As a result, it was confirmed that results are similar to those using conventional Cs₂CO₃. Therefore, it was found that Cs₂CO₃ can be changed to KOH. Additional experiments were conducted to verify the results of Tables 1 and 2 above. It was necessary to check the change in the content of de-isopropylated impurity generated during the work-up process after completion of the reaction and the actual isolation yield as well as the ratio of conversion to isopropylated product in the 5-bromo-3-cyano-1-isopropyl-indole reaction. The contents of the reactions using 7 types of base and acetone as a reaction solvent under reflux condition in the following Reaction Scheme 3 are summarized (Table 3).

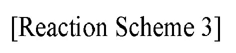

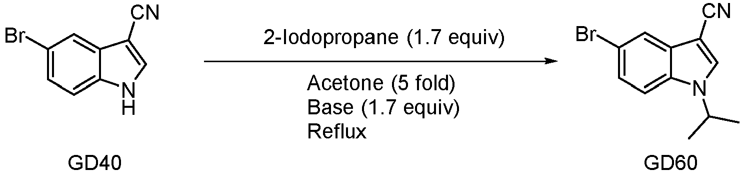

**[Table 3] 5-Bromo-3-cyano-1H-indole isopropylation screening experiment II**

| Run | Base (equiv) | Time (h) | De-isopropylated Impurity (5-bromo-3-cyano-1H-indole) HPLC ratio (%) | | | Gross Yield (%) | Net Yield (%) |
|---|---|---|---|---|---|---|---|
| | | | Before w/up | After w/up | After concentration | | |
| Example 1-3-1 | Cs₂CO₃ | 2.0 | 0.03 | 0.16 | 0.15 | 96.89 | 98.65 |
| Example 1-3-2 | LiOH | 12.0 | 15.37 | 12.76 | 24.80 | 99.83 | 88.41 |
| Example 1-3-3 | Ca(OH)₂ | 24.0 | 99.45 | N/A | N/A | N/A | N/A |
| Example 1-3-4 | NaOH | 5.0 | 0.83 | 0.95 | 0.01 | 84.54 | 85.04 |
| Example 1-3-5 | KOH | 3.0 | 0.12 | 0.29 | 0.01 | 100.97 | 98.76 |
| Example 1-3-6 | K₂CO₃ | 6.0 | 65.03 | N/A | N/A | N/A | N/A |
| Example 1-3-7 | NaOAc | 6.0 | N/A | N/A | N/A | N/A | N/A |

Example 1-3-1 is an experiment using Cs₂CO₃ which was used in the isopropylation reaction, and it was confirmed that de-isopropylated impurity increased from 0.03% to 0.16% when work up and concentration were performed after completion of the reaction. In the case of Example 1-3-2, LiOH was used as a base, and after work up, the organic layer was separated and concentrated, and the sample was analyzed before proceeding with the crystallization process. As a result, it was confirmed that the de-isopropylated impurity increased from 12.76% to 24.80%. In the experiments using NaOH and KOH in Examples 1-3-4 and 1-3-5, the tendency of de-isopropylated impurity to increase during the work up process was also confirmed. Considering the amount of tarr production in the middle layer generated during work up, reaction time, isolation yield and base price, it was concluded that it is preferable to use KOH rather than Cs₂CO₃.

### Example 2: Test results of 5-bromo-3-cyano-1-isopropyl-indole crystallization

### Example 2.1: Process study for 5-bromo-3-cyano-1-isopropyl-indole crystallization

As described in Example 1 above, when the de-isopropylated impurity remains in the process of 5-bromo-3-cyano-1-isopropyl-indole, indole dimer impurity was generated in the C-N coupling reaction of 1-(3-cyano-1-isopropyl-indole-5-yl)pyrazole-4-carboxylic acid ethyl ester, but it was not easy to remove in the later manufacturing process. 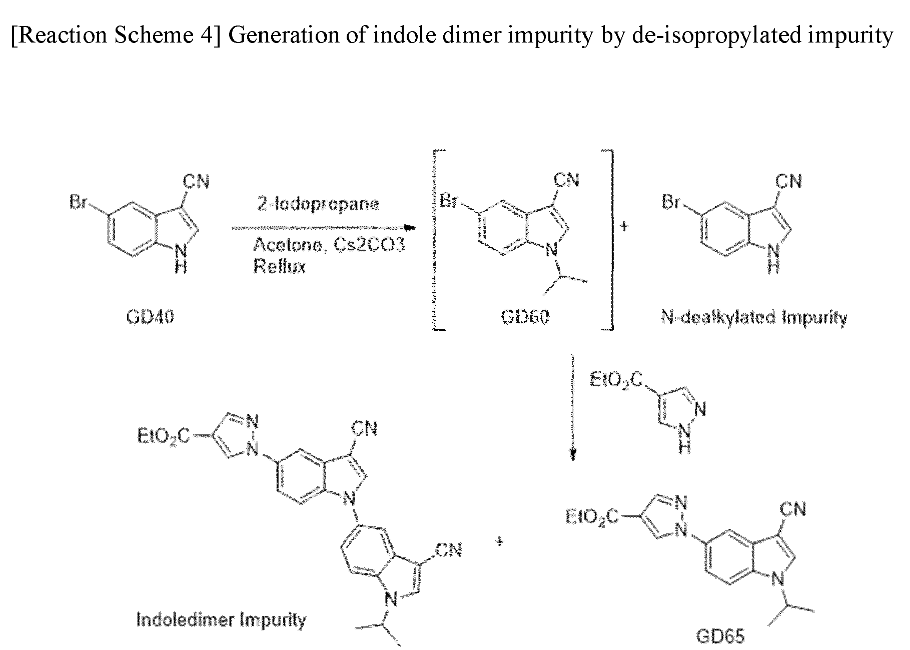
GD40: 5-Bromo-3-cyano-1H-indole
GD60: 5-Bromo-3-cyano-1-isopropyl-indole
GD65: 1-(3-Cyano-1-isopropyl-indol-5-yl)pyrazole-4-carboxylic acid ethyl ester

Specifically, although de-isopropylated impurity was hardly generated during the reaction, the tendency to increase during work up or in the azetropic distillation process using ethyl acetate distillation and toluene was confirmed (Table 4).

**[Table 4] Generation of de-isopropylated impurity**

| Process Step | Result (HPLC % ratio) | |
|---|---|---|
| | 17.6 min (5-bromo-3-cyano-1H-indole, De-isopropylation Impurity) | 21.4 min (5-bromo-3-cyano-1- isopropyl-indole) |
| Final IPC (In Process Control) | n/d | 100 |
| After Work up | 0.538 | 99.462 |
| After Distillation | 3.178 | 96.822 |

As a result of tracking the de-isopropylated impurity of 5-bromo-3-cyano-1-isopropyl-indole by HPLC analysis, it was not detected in the reaction IPC (In Process Control), but was observed to increase during the work up and distillation steps. By using 5-bromo-3-cyano-1-isopropyl-indole obtained as above, the processes of 1-(3-cyano-1-isopropyl-indol-5-yl)pyrazole-4-carboxylic acid ethyl ester, 1-(3-cyano-1-isopropyl-indol-5-yl)pyrazole-4-carboxylic acid and 1-(3-cyano-1-isopropyl-indol-5-yl)pyrazole-4-carboxylic acid were carried out. Then, the final API (active pharmaceutical ingredient) was analyzed. As a result, the impurity expected as an indole dimer was detected, so that the option process was carried out.

### Example 2.1.1. Crystallization of 5-bromo-3-cyano-1-isopropyl-indole using i-PrOH

Based on the results obtained by the solubility curve, crystallization of 5-bromo-3-cyano-1-isopropyl-indole using i-PrOH was performed. In the process of synthesizing 5-bromo-3-cyano-1H-indole using Cs₂CO₃, 5-bromo-3-cyano-1H-indole is regenerated by de-isopropylation of 5-bromo-3-cyano-1-isopropyl-indole after layer separation and participates in 1-(3-cyano-1-isopropyl-indol-5-yl)pyrazole-4-carboxylic acid ethyl ester reaction to generate indole dimer. In order to search for conditions capable of inhibiting the generation of indole dimer by removing 5-bromo-3-cyano-1H-indole in advance, after spiking to comprise 0.86%, 1.56% and 3.00% of 5-bromo-3-cyano-1H-indole, they were dissolved under reflux conditions, cooled slowly at room temperature, kept at 0-5°C for 1 hour, and then filtered. HPLC purity and gross yield of the obtained crystals were summarized (Table 5).

**[Table 5] Residual 5-bromo-3-cyano-1H-indole before and after crystallization of 5-bromo-3-cyano-1-isopropyl-indole using i-PrOH**

| Run | i-PrOH (fold) | Temp. | Time (h) | Result (HPLC % ratio) | | | | Gross Yield (%) |
|---|---|---|---|---|---|---|---|---|
| | | | | Before crystallization | | After crystallization | | |
| | | | | GD40 | GD60 | GD40 | GD60 | |
| Example 2-1-1 | 1.0 | Reflux | 3 | 0.86 | 98.37 | 0.12 | 99.68 | 88 |
| Example 2-1-2 | 1.0 | Reflux | 3 | 1.56 | 96.83 | 0.28 | 99.31 | 87 |
| Example 2-1-3 | 1.0 | Reflux | 3 | 3.00 | 94.10 | 0.59 | 98.63 | 81 |

| | | | | | | | | |
|---|---|---|---|---|---|---|---|---|
| GD40: 5-Bromo-3-cyano-1H-indole GD60: 5-Bromo-3-cyano-1-isopropyl-indole | | | | | | | | |

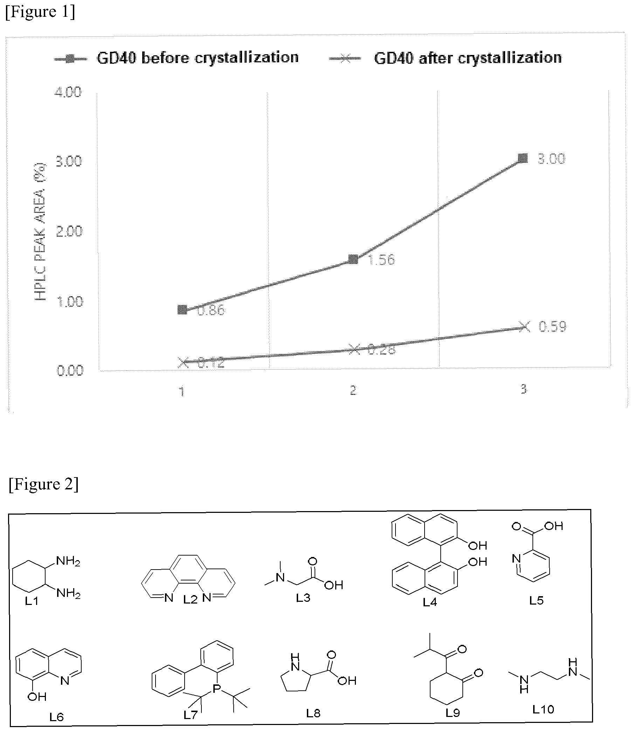

As a result of Examples 2-1-1 to 2-1-3, it was confirmed that a small amount of 5-bromo-3-cyano-1H-indole, which is de-isopropylated impurity, remained even when crystallization was performed by using 1.0-fold of i-PrOH. When the initial starting material 5-bromo-3-cyano-1H-indole was not sufficiently converted to 5-bromo-3-cyano-1-isopropyl-indole, or the content of 5-bromo-3-cyano-1H-indole was increased by increasing the de-isopropylated impurity in the work-up process step, it could not be completely removed even through the crystallization process using 1-fold of i-PrOH was carried out. Figure 1 is a comparison of HPLC peak areas of residual 5-bromo-3-cyano-1H-indole before and after crystallization of 5-bromo-3-cyano-1-isopropyl-indole.

### Example 2.1.2. Crystallization of 5-bromo-3-cyano-1-isopropyl-indole using i-PrOH and anti-solvent (Lab scale)

When crystallization was performed by using i-PrOH, it was confirmed that the residual 5-bromo-3-cyano-1H-indole was purified, and crystallization conditions were searched by using anti-solvents to increase the yield. When crystallization was performed by using i-PrOH, hexane and hetpane, it was confirmed that the residue 5-bromo-3-cyano-1H-indole was effectively removed. There was no significant difference in the net yield between hexane and heptane (Table 6).

**[Table 6] Comparison of residual 5-bromo-3-cyano-1H-indole content before and after crystallization of 5-bromo-3-cyano-1-isopropyl-indole using anti-solvent**

| Run | i-PrOH (fold) | Anti Solvent (fold) | Temp. | Result (HPLC % ratio) | | | | Gross Yield (%) | Net Yield (%) |
|---|---|---|---|---|---|---|---|---|---|
| | | | | Before crystallization | | After crystallization | | | |
| | | | | GD40 | GD60 | GD40 | GD60 | | |
| Example 2-2-1 | 1.0 | Hexane (2.0) | Reflux | 0.68 | 99.32 | 0.00 | 99.99 | 93 | 90 |
| Example 2-1-2 | 1.0 | Hexane (2.0) | Reflux | 1.35 | 98.65 | 0.03 | 99.97 | 93 | 91 |
| Example 2-1-3 | 1.0 | Hexane (2.0) | Reflux | 2.59 | 97.41 | 0.12 | 99.88 | 93 | 92 |
| Example 2-1-4 | 1.0 | Heptane (2.0) | Reflux | 0.71 | 99.29 | 0.02 | 99.98 | 94 | 92 |
| Example 2-1-5 | 1.0 | Heptane (2.0) | Reflux | 1.38 | 98.62 | 0.06 | 99.94 | 94 | 92 |
| Example 2-1-6 | 1.0 | Heptane (2.0) | Reflux | 2.54 | 97.46 | 0.12 | 99.88 | 94 | 93 |

| | | | | | | | | | |
|---|---|---|---|---|---|---|---|---|---|
| GD40: 5-Bromo-3-cyano-1H-indole GD60: 5-Bromo-3-cyano-1-isopropyl-indole | | | | | | | | | |

### Example 2.1.3. Search for crystallization conditions of 5-bromo-3-cyano-1-isopropyl-indole

Additional tests were performed to determine whether 5-bromo-3-cyano-1H-indole was removed while improving the yield in the crystallization process of 5-bromo-3-cyano-1-isopropyl-indole. After spiking of 0.78% and 2.59%, and crystallization, the amount of residual 5-bromo-3-cyano-1H-indole was evaluated by HPLC.

When the amount of i-PrOH was reduced, it was confirmed that the yield of 5-bromo-3-cyano-1-isopropyl-indole was improved, but 5-bromo-3-cyano-1H-indole remained. In view of these results, in order to completely remove the residual 5-bromo-3-cyano-1H-indole, it seemed preferable to proceed with the crystallization under the condition that 1-fold of i-PrOH is used and 2-fold of heptane is used. However, if crystallization is carried out in a state in which 5-bromo-3-cyano-1H-indole is present in an amount of 0.78% or less during the reaction, it was determined that it would be manageable even if crystallization was performed by using 0.5-fold of i-PrOH (Table 7).

**[Table 7] Crystallization condition result of 5-bromo-3-cyano-1-isopropyl-indole through i-PrOH ratio change**

| Run | i-PrOH (fold) | Heptane (fold) | Temp. | Result (HPLC % ratio) | | | | Gross Yield (%) |
|---|---|---|---|---|---|---|---|---|
| | | | | Before crystallization | | After crystallization | | |
| | | | | GD40 | GD60 | GD40 | GD60 | |
| Example 2-3-1 | 1.0 | 2.0 | Reflux | 0.71 | 99.39 | 0.00 | 99.99 | 93 |
| Example 2-3-2 | - | 2.0 | Reflux | 0.78 | 99.42 | 0.68 | 99.99 | 95 |
| Example 2-3-3 | 0.25 | 2.0 | Reflux | 0.78 | 99.42 | 0.11 | 99.89 | 95 |
| Example 2-3-4 | 0.25 | 2.0 | Reflux | 2.59 | 99.42 | 0.17 | 99.83 | 94 |
| Example 2-3-5 | 0.50 | 2.0 | Reflux | 0.78 | 97.41 | 0.04 | 99.94 | 94 |
| Example 2-3-6 | 0.50 | 2.0 | Reflux | 2.59 | 97.41 | 0.14 | 99.86 | 94 |

| | | | | | | | | |
|---|---|---|---|---|---|---|---|---|
| GD40: 5-Bromo-3-cyano-1H-indole GD60: 5-Bromo-3-cyano-1-isopropyl-indole | | | | | | | | |

### Example 3: Preparation method of 5-bromo-3-cyano-1-isopropyl-indole

Acetone (800 L), 5-bromo-3-cyano-1H-indole (300 kg), KOH (114 kg) and 2-iodopropane (346 kg) were sequentially put into a reactor, and the reaction was carried out under elevated temperature and reflux conditions. After confirming the completion of the reaction by HPLC, the reaction mixture was cooled to room temperature. For extraction and layer separation, EtOAc (1,353 kg) and purified water (1,500 kg) were additionally added to the reaction mixture, stirred for 1 hour, kept for 1 hour, and the aqueous layer formed below was discarded. The remaining organic layer was micro-filtrated and concentrated as much as possible by distillation, and residual EtOAc was checked by GC. After adding 2-propanol (150 L) and heptane (816 kg) to the concentrated reaction mixture, the temperature was raised to 78°C. When it was visually confirmed that the reaction mixture was clear, the reaction mixture was additionally stirred for 30 minutes and then slowly cooled down. Cooling was carried out over about 6 hours, and filtration was performed after keeping at 5-10°C for 1 hour. The filtered solid was washed with heptane (408 kg) and dried with nitrogen to obtain 5-bromo-3-cyano-1-isopropyl-indole (335.7 kg, 94.0% gross yield).

### Example 4: Test results of 1-(3-cyano-1-isopropyl-indol-5-yl)pyrazole-4-carboxylic acid ethyl ester reaction

### Example 4.1. Process study for 1-(3-cyano-1-isopropyl-indol-5-yl)pyrazole-4-carboxylic acid ethyl ester

In the existing preparation process of 1-(3-cyano-1-isopropyl-indol-5-yl)pyrazole-4-carboxylic acid ethyl ester, concentrated crude 5-bromo-3-cyano-1-isopropyl-indole, 1H-pyrazole-4-carboxylic acid ethyl ester, CuI as a catalyst, 1,2-cyclohexanediamine (1,2-CHDA) as a ligand, K₂CO₃ as a base and toluene as a reaction solvent were used, and the reaction mixture was stirred for 38-45 hours under reflux condition, cooled and proceeded to work up using NH₄OH and purified water. After work up, the layer-separated organic layer included excess tar and solid impurities. To remove these impurities, Na₂SO₄ and silica gel were added, stirred and then filtered. At this time, the amount of Na₂SO₄ and silica gel was used in the same weight ratio as 5-bromo-3-cyano-1-isopropyl-indole, so that a large amount of solid waste was generated in the filtration process step. The filtrate was transferred to a washed reactor and concentrated, and after concentration was complete, i-PrOH was added to perform a crystallization process through temperature increase and cooling to obtain 1-(3-cyano-1-isopropyl-indol-5-yl)pyrazole-4-carboxylic acid ethyl ester. The average yield of the existing preparation process was about 56%. 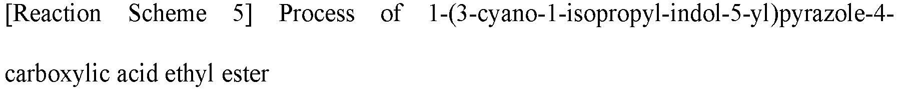 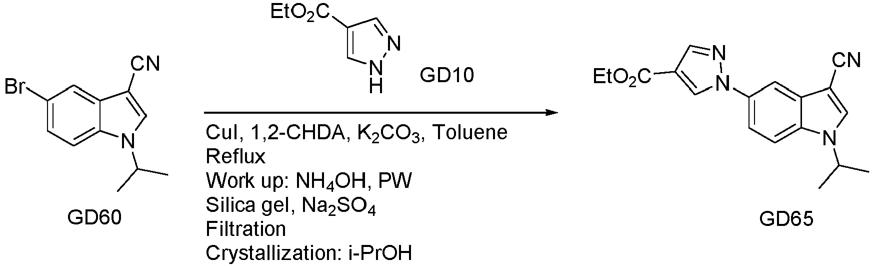
GD10: 1H-pyrazole-4-carboxylic acid ethyl ester
GD60: 5-Bromo-3-cyano-1-isopropyl-indole
GD65: 1-(3-Cyano-1-isopropyl-indol-5-yl)pyrazole-4-carboxylic acid ethyl ester

### Example 4.1.1. Ligand screening for 1-(3-cyano-1-isopropyl-indol-5-yl)pyrazole-4-carboxylic acid ethyl ester reaction

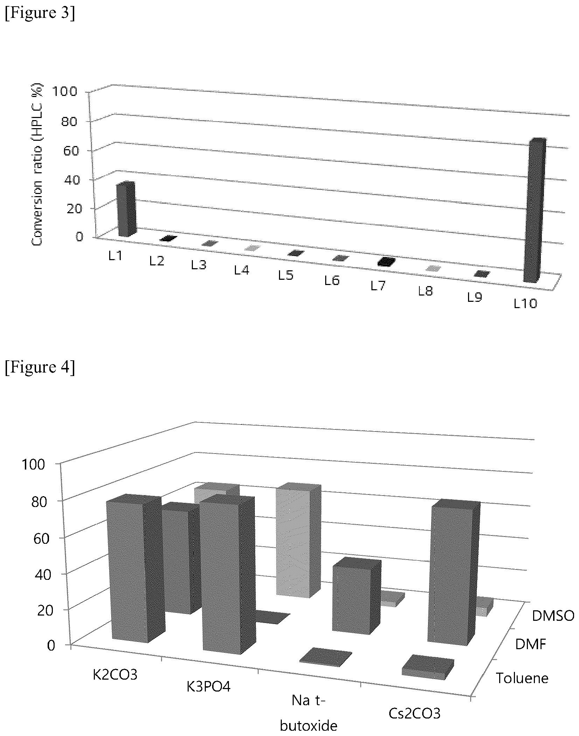

As in the above process, in the case of 1-(3-cyano-1-isopropyl-indol-5-yl)pyrazole-4-carboxylic acid ethyl ester, 1,2-CHDA (1,2-cyclohexanediamine, L1) was used for the C-N coupling reaction. In this case, it was confirmed that the primary amine ligand is coupled with 5-bromo-3-cyano-1-isopropyl-indole, resulting in excessive 5-bromo-3-cyano-1-isopropyl-indole-ligand impurity. Eventually, the impurity coupled with the ligand not only affects the yield, but also delays the completion of the reaction or the reaction is not completed. In order to solve these problems, ligand screening was performed first. Ligands used for screening were tested by selecting N-N ligands, N-O ligands and other applicable ligands (Figure 3).

Ligand screening experimental conditions were as follows: 5-bromo-3-cyano-1-isopropyl-indole (1.0 equiv), 1H-pyrazole-4-carboxylic acid ethyl ester (1.0 equiv), toluene (4 fold, fold = ml/g of 5-bromo-3-cyano-1-isopropyl-indole), CuI (0.2 equiv), K₂CO₃ (2,0 equiv) and ligand (0.4 equiv) were added and stirred at external set temperature of 125-130°C for 24 hours, and the conversion ratio (%) of 5-bromo-3-cyano-1-isopropyl-indole to 1-(3-cyano-1-isopropyl-indol-5-yl)pyrazole-4-carboxylic acid ethyl ester was used as the evaluation standard (Figure 3).

As can be seen from Figure 3, when the ligand L1 (1,2-cyclohexanediamine) was used, the conversion rate was 38.85%, whereas when the ligand L10 (N,N-dimethylethylenediamine, DMEDA) was used, the high conversion rate of 87.28% was confirmed.

Eventually, if the ligand is changed to L10 at the same reaction time, the reaction rate is improved and impurity is suppressed, resulting in a higher yield. Except for L2 (1,2-phenanthroline) 0.41% and L7 (JohnPhos) 2.1%, no reaction proceeded at all in the case of the other ligands.

### Example 4.1.2. Base and solvent screening for 1-(3-cyano-1-isopropyl-indol-5-yl)pyrazole-4-carboxylic acid ethyl ester reaction

In Example 4.1.1. it was confirmed the ratio of converting to 1-(3-cyano-1-isopropyl-indol-5-yl)pyrazole-4-carboxylic acid ethyl ester is increased in the case of using the L10 ligand rather than the previously used L1 ligand. Next, a screening experiment for the base was performed (Figure 4).

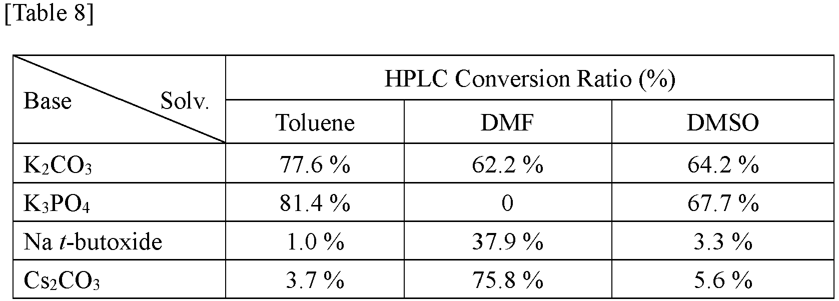

The experimental conditions of Figure 4 were as follows: 5-bromo-3-cyano-1-isopropyl-indole (1.0 equiv), 1H-pyrazole-4-carboxylic acid ethyl ester (1.0 equiv), solvent (4 fold, fold = ml/g of 5-bromo-3-cyano-1-isopropyl-indole), CuI (0.2 equiv), base (2.0 equiv) and 1,2-CHDA (0.4 equiv) were added and stirred at the external set temperature of 125-130°C for 24 hours, and the experiment was conducted with the conversion ratio (%) of 5-bromo-3-cyano-1-isopropyl-indole to 1-(3-cyano-1-isopropyl-indol-5-yl)pyrazole-4-carboxylic acid ethyl ester as the evaluation standard. First, as the results of the reactions using K₂CO₃, K₃PO₄, Na t-butoxide and Cs₂CO₃ as a base in toluene solvent, the conversion rates of 77.6% and 81.4% were confirmed in K₂CO₃ and K₃PO₄. On the other hand, almost no reaction proceeded with Na t-butoxide and Cs₂CO₃. When the reaction solvent was changed to DMF, K₂CO₃, Na t-butoxide and Cs₂CO₃ showed good conversion compared to that using toluene, and K₃PO₄ did not react at all. When DMSO was used, it showed a similar tendency to the experiment using toluene, but the conversion rate was relatively low. In the above experiments, it was determined that it is preferable to use K₂CO₃ as the base and toluene as the solvent in consideration of the purchase price of the base and solvent, the safety of the preparation process, and the latter process (work up and distillation).

### Example 4.1.3. Catalyst screening for 1-(3-cyano-1-isopropyl-indol-5-yl)pyrazole-4-carboxylic acid ethyl ester reaction

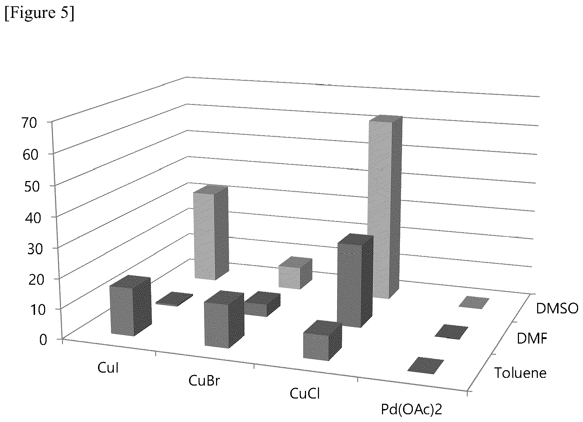

In Example 4.1.1. and Example 4.1.2., it was determined that the reaction rate and conversion rate are excellent when N,N-dimethylethylenediamine (L10) is used as a ligand rather than 1,2-cyclohexanediamine (L1), and K₂CO₃ and toluene as the base and reaction solvent are preferable for 1-(3-cyano-1-isopropyl-indol-5-yl)pyrazole-4-carboxylic acid ethyl ester reaction. Because the synthesis of 1-(3-cyano-1-isopropyl-indol-5-yl)pyrazole-4-carboxylic acid ethyl ester is Ullmann reaction in which a copper source is used as a catalyst for C-N coupling of 1H-pyrazole-4-carboxylic acid ethyl ester and 5-bromo-3-cyano-1-isopropyl-indole, it was necessary to screen various copper catalysts. In addition, it was necessary to study the cross-coupling reaction of the Buchwald-Hartwig type using a palladium catalyst. The screening experiments for a total of four catalysts were performed (Figure 5).

Experimental conditions were as follows: 5-bromo-3-cyano-1-isopropyl-indole (1.0 equiv), 1H-pyrazole-4-carboxylic acid ethyl ester (1.0 equiv), solvent (4 fold, fold = ml/ g of 5-bromo-3-cyano-1-isopropyl-indole), catalyst (0.2 equiv), K₂CO₃ (2.0 equiv) and 1,2-CHDA (0.4 equiv) were added and stirred at the external set temperature of 125-130°C for 24 hours, and the experiment was conducted with the conversion ratio (%) of 5-bromo-3-cyano-1-isopropyl-indole to 1-(3-cyano-1-isopropyl-indol-5-yl)pyrazole-4-carboxylic acid ethyl ester was used as the evaluation standard.

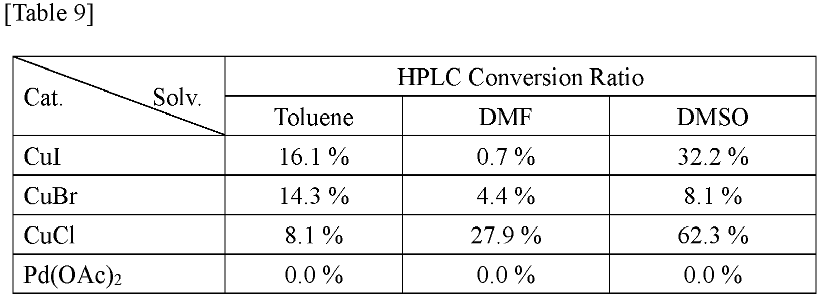

In the case of the catalyst screening experiments, a carousel multi reactor was used. According to the experimental results, although CuCl showed the highest conversion ratio of 5-bromo-3-cyano-1-isopropyl-indole to 1-(3-cyano-1-isopropyl-indol-5-yl)pyrazole-4-carboxylic acid ethyl ester under DMSO reaction solvent, debrominated impurity could be detected by HPLC as a side reaction. Although Pd(OAc)₂ was used as the palladium source, the reaction did not proceed at all in the three reaction solvents. In the case of Cul, debrominated impurity could be detected when DMSO solvent was used. As a result, when the reaction was carried out by using CuCl and CuI in DMSO solvent, the conversion rate of 5-bromo-3-cyano-1-isopropyl-indole to 1-(3-cyano-1-isopropyl-indol-5-yl)pyrazole-4-carboxylic acid ethyl ester was high, but it was confirmed that there was a problem that the production amount of debrominated impurity increased at the same time. Therefore, it was concluded that DMSO solvent was not suitable for the reaction. Through the above experiments, it was confirmed that the conversion rate was higher when CuI was used as a catalyst in the toluene reaction solvent than when CuBr, CuCl, and Pd(OAc)₂ were used. Finally, toluene was selected as the reaction solvent, and CuI was selected as the catalyst.

### Example 5: Test result of removing residual Cu in 1-(3-cyano-1-isopropyl-indol-5-yl)pyrazole-4-carboxylic acid ethyl ester

A new approach was needed to improve the 1-(3-cyano-1-isopropyl-indol-5-yl)pyrazole-4-carboxylic acid ethyl ester work up process. Specifically, a new method in which the adsorption process is omitted and the layer separation can be effectively performed was considered. Eventually, there was a need to increase the amount of purified water used to effectively remove Cu-complex, KBr, K₂CO₃, excess tar and other by-products, and a method to effectively remove copper by chelating was required. For this purpose, work up was performed using citric acid and EDTA (ethylenediaminetetraacetic acid), which are chelating agents.

### Example 5.1. 1-(3-Cyano-1-isopropyl-indol-5-yl)pyrazole-4-carboxylic acid ethyl ester work up study (II)

### Example 5.1.1. Work up study (citric acid)

When performing work up after the reaction in the toluene solvent, the organic layer and the aqueous layer became a turbid lump, and the layers were not separated well, so after adding ethyl acetate, work up was carried out. Among the various work up methods to replace NH₄OH, when 10% citric acid solution was used, the aqueous layer was clean and the layers were separated well, so it was decided to use 10% citric acid solution for the first work up.

Then, after adding purified water, the pH was lowered to 2-3 using 3N HCl to clearly break the Cu complex. The next step was the treatment with 5% EDTA disodium solution to remove the remaining Cu, and this process was repeated once more. Finally, purified water was added to wash off any remaining salts (Schematic Diagram 1). 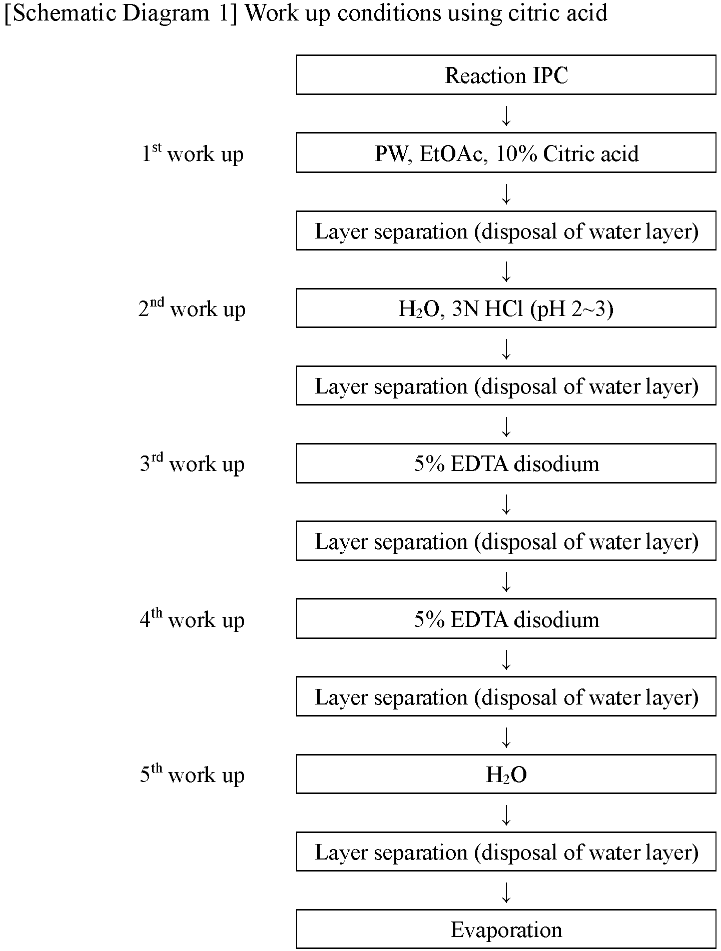
* Each of the solutions was added by 6-fold.

### Example 5.1.2. Work up optimization (10% citric acid + HCl)

When the solution used for the first work up was combined with aq. HCl, it was attempted to find the optimal ratio showing good layer separation behavior (Table 10). Based on these results, it was determined that the optimal condition is to use 2-fold of 10% citric acid aqueous solution and 2-fold of 6N HCl aqueous solution, respectively, at the same time, and the appropriate pH range was 2.75 - 3.5.

**[Table 10] Conditions and results of the first work up ^{a)}**

| Run | Work up solution | Fold | HCl | Fold | pH | Layer separation appearance |
|---|---|---|---|---|---|---|
| Example 5-1-1 | 10% NH₄Cl aqueous solution | 3 | 3N | 1 | 8.12 | Cloudy aqueous layer |
| Example 5-1-2 | 10% NH₄Cl aqueous solution | 2 | 3N | 2 | 6.94 | Cloudy aqueous layer |
| Example 5-1-3 | 10% NH₄Cl aqueous solution | 1 | 3N | 3 | 6.85 | Very cloudy aqueous layer |
| Example 5-1-4 | 10% citric acid aqueous solution | 3 | 3N | 1 | 7.4 | Good layer separation |
| Example 5-1-5 | 10% NH₄Cl aqueous solution | 3 | 6N | 1 | 8.1 | Cloudy aqueous layer |
| Example 5-1-6 | 10% citric acid aqueous solution | 3 | 6N | 1 | 8.5 | Aqueous layer is full of bubbles |
| Example 5-1-7 | 10% NH₄Cl aqueous solution | 4 | 6N | 2 | 8.1 | Bad layer separation |
| Example 5-1-8 | 10% citric acid aqueous | 4 | 6N | 2 | 3.5 | Good layer separation, Easy to distinguish |
| | solution | | | | | color |
| Example 5-1-9^{b)} | 10% NH₄Cl aqueous solution | 8 | - | - | - | Aqueous layer is full of bubbles |
| Example 5-1-10 | 10% citric acid aqueous solution | 2 | 6N | 2 | 2.75 | Good layer separation, Easy to distinguish color |
| Example 5-1-11 | 10% citric acid aqueous solution | 2.5 | 6N | 1.5 | 4.70 | Good layer separation, Easy to distinguish color, Dark organic layer |
| Example 5-1-12 | 10% citric acid aqueous solution | 2.2 | 6N | 1.8 | 3.72 | Good layer separation, Easy to distinguish color, Dark organic layer |

| | | | | | | |
|---|---|---|---|---|---|---|
| a) The reaction was carried out on a 10.0 g scale, b) The reaction was carried out on a 100.0 g scale | | | | | | |

Then, a scale-up experiment was performed (Table 11). In a 100.0 g scale using a 2 L reactor, when 4-fold of 10% aqueous citric acid solution was added, 2.8-fold of 6N HCl was added, and when 8-fold of 10% citric acid aqueous solution was added, 2.2-fold of 6N HCl was added, layer separation was good and color distinguishing was easy.

**[Table 11] Conditions and results of the first work up**

| Run | Scale | 10% Citric acid | 6N HCl | Layer separation appearance |
|---|---|---|---|---|
| Example 5-2-1 | 100.0 g | 2 fold | 2 fold | The presence of floating matter in the aqueous layer. The color of the aqueous layer is not yet changed to green. |
| Example 5-2-2 | 100.0 g | 2 fold | 3 fold | The color of the organic layer becomes very dark. Layer separation is good, but there is floating matter in the aqueous layer in the next step. |
| Example 5-2-3 | 20.0 g | 4 fold | 2.8 fold | Good layer separation, Easy to distinguish color |
| Example | 20.0 g | 8 fold | 2.2 fold | Good layer separation, Easy to distinguish |
| 5-2-4 | | | | color |
| Example 5-2-5 | 100.0 g | 4 fold | 2.8 fold | Good layer separation, Easy to distinguish color |
| Example 5-2-6 | 100.0 g | 4 fold | 2.5 fold | Good layer separation, Easy to distinguish color |

Conventionally, Cu was removed through a process of filtering after adding silica gel and Na₂SO₄, but a large amount of solid waste was generated in this process, and a lot of time was required to treat this solid waste. In order to solve this problem, as a result of conducting various experiments shown in Example 5, a new work up method was discovered and the filtration process could be replaced with a work up process utilizing the chelation principle.

### Example 6: Preparation of 1-(3-cyano-1-isopropyl-indol-5-yl)pyrazole-4-carboxylic acid ethyl ester

Toluene (880 L), 5-bromo-3-cyano-1-isopropyl-indole (220 kg), 1H-pyrazole-4-carboxylic acid ethyl ester (129 kg) and K₂CO₃ (231 kg) were added to a reactor, and N₂ purge was performed for about 30 minutes. After adding CuI (32 kg) and DMEDA (30 kg), the internal temperature was raised to 45°C while maintaining the N₂ purge. After 9 hours of reaction under reflux condition, reaction IPC (in process control) was performed to confirm the completion of the reaction, and the reaction mixture was cooled to room temperature. The reaction time of 35 hours in the existing process was reduced to 9 hours. For extraction and layer separation, 10% aqueous citric acid solution (880 L) and EtOAc (794 kg) were sequentially added to the reaction mixture, and then 6 N HCl (381 kg) was added dropwise to adjust pH = 2-3. After stirring for 30 minutes, the resulting product was allowed to stand for 1 hour, and the separated aqueous layer was discarded. After adding 5% EDTA aqueous solution (880 L) to the remaining organic layer, it was stirred for 30 minutes and allowed to stand for 1 hour to proceed with layer separation. The same process was repeated twice in total. Finally, after adding purified water (880 L), the aqueous layer separated by stirring and standing for 30 minutes was discarded. In the above processes, the extraction and layer separation were performed a total of 4 times at about 35°C. The remaining organic layer was distilled as much as possible after microfiltration, and concentrated IPC (in process control) was performed to confirm that EtOAc was removed. After the distillation was completed, 2-propanol (880 L) was added to the reaction mixture, and then the temperature was raised. When it was confirmed that the reaction mixture was clear, it was cooled slowly to 0-10°C for 7-8 hours, kept for about 1 hour and filtered. The filtered solid was washed with 2-propanol (880 L) and dried using nitrogen and vacuum to obtain 1-(3-cyano-1-isopropyl-indol-5-yl)pyrazole-4-carboxylic acid ethyl ester (253.5 kg, 94.0% gross yield). This is about 1.7 times higher than 56% yield of the existing process.

## Claims

1. A method for preparing a compound of the following Formula 2, comprising:
i) a step of reacting a compound of Formula 1 and X-R3 with a base comprising KOH in an organic solvent; and
ii) a step of crystallizing a product obtained in step (i) by using alcohol and an anti-solvent comprising hydrocarbon having 5 to 8 carbon atoms: 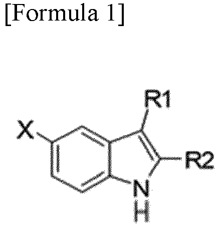 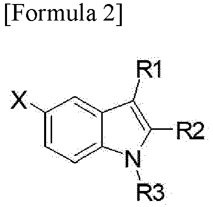 wherein,
X is F, Cl, Br or I;
R1 is hydrogen or CN;
R2 is hydrogen, halogen, C₁-C₇ alkyl, C₁-C₇ alkoxy-C₁-C₇ alkyl or phenyl; and
R3 is hydrogen; C₁-C₇ alkyl unsubstituted or substituted by one or more substituents selected from halogen, C₃-C₇ cycloalkyl and O-R6; C₃-C₇ cycloalkyl; or 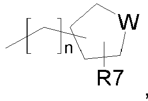 wherein R6 is C₁-C₄ alkyl, W is O or S, R7 is hydrogen or C₁-C₄ alkyl, and n is an integer of 0 to 3.

2. The method according to Claim 1, wherein X-R3 is 2-iodopropane.

3. The method according to Claim 1, wherein the organic solvent is acetone.

4. The method according to Claim 1, wherein the alcohol is one or more selected from the group consisting of methanol, ethanol, propanol, isopropanol and butanol.

5. The method according to Claim 1, wherein the hydrocarbon having 5 to 8 carbon atoms is selected from the group consisting of hexane, heptane and a mixture thereof.

6. A method for preparing a compound of the following Formula 4, comprising:
a step of carrying out a C-N coupling reaction of a compound of Formula 2 and a compound of Formula 3 with a copper catalyst, a base and a ligand comprising N,N-dimethylethylenediamine in an organic solvent: 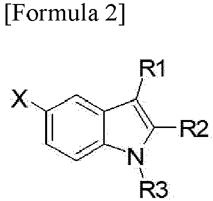 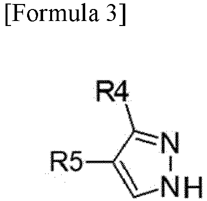 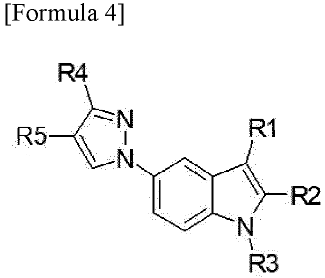
wherein,
X is F, Cl, Br or I;
R1 is hydrogen or CN;
R2 is hydrogen, halogen, C₁-C₇ alkyl, C₁-C₇ alkoxy-C₁-C₇ alkyl or phenyl;
R3 is hydrogen; C₁-C₇ alkyl unsubstituted or substituted by one or more substituents selected from halogen, C₃-C₇ cycloalkyl and O-R6; C₃-C₇ cycloalkyl; or 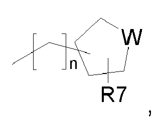 wherein R6 is C₁-C₄ alkyl, W is O or S, R7 is hydrogen or C₁-C₄ alkyl, and n is an integer of 0 to 3;
R4 is hydrogen, halogen or C₁-C₇ alkyl; and
R5 is -C(O)OR8, wherein R8 is hydrogen, C₁-C₇ alkyl or C₃-C₇ cycloalkyl.

7. The method according to Claim 6, wherein the organic solvent is one or more selected from the group consisting of xylene, toluene, dimethylformamide (DMF) and dimethyl sulfoxide (DMSO).

8. The method according to Claim 6, wherein the copper catalyst is one or more selected from the group consisting of CuI, Cu(OAc)₂, Cu, Cu₂O and CuO.

9. The method according to Claim 6, wherein the base is one or more selected from the group consisting of potassium carbonate, cesium carbonate, potassium phosphate tribasic, triethylamine and sodium tert-butoxide.

10. The method according to Claim 6, which further comprises a step of purifying the compound of Formula 4 by using:
one or more chelating agents selected from the group consisting of EDTA, citric acid, potassium citrate and sodium citrate; and
one or more ligand reagents selected from the group consisting of ammonium chloride and aqueous ammonia.

11. The method according to any one of Claims 1 to 5, wherein R1 is CN, R2 is hydrogen, and R3 is isopropyl.

12. The method according to any one of Claims 6 to 10, wherein R1 is CN, R2 is hydrogen, R3 is isopropyl, R4 is hydrogen, and R5 is ethoxycarbonyl (-C(O)OEt).
